# EUROPEAN PATENT SPECIFICATION

(11) **EP 3 182 133 B1**
(45) Date of publication and mention of the grant of the patent: **15.01.2020**
(21) Application number: 15200580.7
(22) Date of filing: 16.12.2015
(51) Int. Cl.: B01L 3/00, G01N 35/00, G06F 17/00

(54) **AUTOMATED ANALYTICAL SYSTEM AND METHOD FOR OPERATING THEREOF**
AUTOMATISIERTE ANALYTISCHE SYSTEME UND VERFAHREN ZUM BETRIEB DAVON
SYSTÈME ANALYTIQUE AUTOMATISÉ ET PROCÉDÉ POUR SON FONCTIONNEMENT

(43) Date of publication of application: 21.06.2017
(73) Proprietor: F. Hoffmann-La Roche AG, 4070 Basel (CH); Roche Diagnostics GmbH, 68305 Mannheim (DE)
(72) Inventor: Colpaert, Filip, 9800 Deinze (BE); Schindler, Florian, 6343 Rotkreuz (CH)
(74) Representative: Gyenge, Zoltán

(56) References cited:
- EP-A1- 2 518 514
- WO-A1-94/22580
- US-A- 3 831 006
- US-A- 4 645 916
- US-A- 5 164 575
- US-A1- 2015 031 143

## Description

### TECHNICAL FIELD

The application relates to a laboratory system and a method for operating a laboratory system which is operable to process biological samples.

### BACKGROUND AND RELATED ART

In vitro diagnostic testing has a major effect on clinical decisions, providing physicians with pivotal information.

In analytical laboratories, in particular clinical laboratories, a multitude of analyses on samples are executed by an analytical system in order to determine the physiological state of a patient. Current pre-analytical instruments on the market are able to prepare a plurality of samples such as blood, urine, cerebral-spinal fluid, saliva etc. Biological samples are typically contained in open or capped sample tubes. Before a chemical, biological, physical, optical or other technical analysis can be performed on a sample, a plurality of different pre-analytical processing steps may have to be executed on a sample of a patient. Such processing steps may comprise centrifugation, resuspension (e.g. by mixing or vortexing), capping, decapping, recapping and/or aliquotation steps. Said processing steps may also comprise adding chemicals or buffers to a sample, concentrating a sample, incubating a sample, and the like. A growing number of those 'pre-analytical' steps and procedures are executed automatically by automated pre-analytical instruments, also known as automated pre-analytical systems'.

The kind of analytical test to be executed on a biological sample is typically specified in a test order which is typically registered in a laboratory information system and sent to the laboratory system.

However, the physical transport and processing of a sample tube may not always be in synchronism with the assignment of a particular analytical test to said sample. For example, a blood sample may be drawn from a patient, collected in a sample tube and transported manually or automatically to an instrument of a laboratory system at a moment in time when it is not clear which kind of biological and/or chemical analysis, also referred to as 'analytical test' or 'assay' shall be executed on said sample. A physician may have to conduct additional examinations of the patient before he can decide which kind of analytical test should be executed on the blood sample of the patient. While the physician conducts said additional examinations, the blood sample of the patient may already arrive at the laboratory instrument, leaving the instrument with the question what to do with the sample. According to some laboratory settings a plurality of samples is received together with a pile of paper-based test orders. In said scenarios, the assignment of electronic test orders to samples may be delayed as the data on the paper-based test orders needs to be entered manually into the software of a host system (such as a Laboratory Information System LIS) before an electronic test order can be requested for a particular sample.

Most state-of-the-art laboratory systems are not operable to prepare a sample for a particular analysis as long as a test order for said sample is not available. Valuable time is lost, as said laboratory instrument is not able to process that sample at all or at best it is merely able to carry samples not having assigned a test order to a buffering station.

If a test order is not available for a sample, some state-of-the-art laboratory systems determine the type of the sample tubes of a sample and process said sample based on its tube type. For example, known laboratory instruments determine the sample container type by means of an image analysis for distributing the containers to different areas or racks. EP 2 518 514 presents such a laboratory instrument. While in many cases such an approach is advantageous, one disadvantage of this approach is that information on the tube type alone is often not sufficient to determine all processing steps necessary to prepare a particular sample for a particular analytical test. In addition, said systems may not be suitable in a lab there may exist much more processing workflows and corresponding test orders than tube types.

### SUMMARY

The laboratory system and method for operating a laboratory system as defined in the independent claims, aim to enable the processing of biological samples even in the absence of a definite test order to avoid delays in sample processing from the time a sample is received by the laboratory system until a definite test order corresponding to that sample becomes available.

According to particular embodiments of the disclosed laboratory system / method, a provisional test order is generated for samples with no corresponding definite test order to bridge the time span until a test order is registered, the laboratory system being instructed to execute processing steps of a provisional test order as soon as possible after receipt of a sample. According to particular embodiments of the disclosed laboratory system / method, processing step(s) of a definite test order (from a host such as a LIS) and provisional processing steps of the provisional test order are consolidated as soon as the definite test order becomes available. Thereafter, according to further embodiments of the disclosed laboratory system / method, the laboratory system is instructed to cancel execution of not yet executed provisional processing steps of the provisional test order and to execute the remainder processing step(s) of the definite test order, if the provisional processing steps of the provisional test order not yet executed conflict with the remainder processing steps of the definite test order.

According to particular embodiments of the disclosed laboratory system / method, generating the provisional test order is based on matching a template over the sample tube identifier and/or identifying an interval to which the sample tube identifier belongs and/or determining the tube type of the sample tube that contains said one or more biological sample followed by retrieving one or more provisional processing steps to be carried out on biological samples identified by sample tube identifiers matching the template, respectively within the identified interval or biological samples received in sample tubes of the determined tube type.

According to particular embodiments of the disclosed laboratory system / method, the provisional processing steps of the provisional test order are executed on an aliquot of the biological sample and wherein all the processing steps of the definite test order are carried out on the biological sample if one or more of the executed provisional processing steps of the provisional test order executed by the laboratory system on the biological sample conflicts with one or more processing steps of the definite test order.

### BRIEF DESCRIPTION OF THE DRAWINGS

Further characteristics and advantages of the disclosed method / device /system will in the following be described in detail by means of the description and by making reference to the drawings. Which show:
- Fig. 1: a highly schematic block diagram of an embodiment of the disclosed laboratory system;
- Fig. 2: a flowchart illustrating an embodiment of the disclosed method for operating a laboratory system;
- Fig. 3: a flowchart illustrating a further embodiment of the disclosed method for operating a laboratory system;
- Fig. 4: a flowchart illustrating an even further embodiment of the disclosed method for operating a laboratory system;
- Fig. 5: a flowchart illustrating an even further embodiment of the disclosed method for operating a laboratory system;
- Fig. 6: a flowchart illustrating an even further embodiment of the disclosed method for operating a laboratory system;
- Fig. 7A: an illustration of execution of processing steps, illustrating the cancelation of the execution of provisional processing steps of a provisional test and handover to the execution of remainder processing steps of a definite test order;
- Fig. 7B: a further illustration of execution of processing steps, illustrating raising of an alert if executed provisional processing steps of a provisional test order conflict with processing steps of a definite test order;
- Fig. 8: a schematic illustration of an embodiment of the disclosed laboratory system, wherein the sample input station is comprised within a pre-analytical laboratory instrument ;
- Fig. 9: a schematic illustration of an embodiment of the disclosed laboratory system; wherein the sample input station is a dedicated laboratory instrument for loading samples;
- Fig. 10: a schematic illustration of an embodiment of the disclosed laboratory system integrated into a single laboratory instrument.

Note: The figures are not drawn to scale, are provided as illustration only and serve only for better understanding but not for defining the scope of the invention. No limitations of any features of the invention should be inferred from these figures.

### DETAILED DESCRIPTION

Certain terms will be used in this patent application, the formulation of which should not be interpreted to be limited by the specific term chosen, but as to relate to the general concept behind the specific term.

The term **'laboratory instrument'** as used herein encompasses any apparatus or apparatus component operable to execute one or more processing steps / workflow steps on one or more biological samples. The expression 'processing steps' thereby refers to physically executed processing steps such as centrifugation, aliquotation, sample analysis and the like. The term 'laboratory instrument' covers pre-analytical instruments, post-analytical instruments and also analytical instruments.

The term **'pre-analytical instrument'** as used herein comprises one or more lab-devices for executing one or more pre-analytical processing steps on one or more biological samples, thereby preparing the samples for one or more succeeding analytical tests. A pre-analytical processing step can be, for example, a centrifugation step, a capping-, decapping- or recapping step, an aliquotation step, a step of adding buffers to a sample and the like. The expression **'analytical system'** as used herein encompasses any monolithic or multi-modular laboratory device comprising one or more lab-devices or operative units which are operable to execute an analytical test on one or more biological samples.

The term **'post-analytical instrument'** as used herein encompasses any laboratory instrument being operable to automatically process and/or store one or more biological samples. Post-analytical processing steps may comprise a recapping step, a step for unloading a sample from an analytical system or a step for transporting said sample to a storage unit or to a unit for collecting biological waste.

The term **'analyzer' / 'analytical instrument'** as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term **'laboratory system'** as used herein encompasses any system for the use in a laboratory comprising one or more laboratory instrument(s) operatively connected to a control unit.

The term '**control unit**' as used herein encompasses any physical or virtual processing device configurable to control a laboratory system comprising one or more laboratory instruments in a way that workflow(s) and workflow step(s) are conducted by the laboratory system. The control unit may, for example, instruct the laboratory system (or a specific instrument thereof) to conduct pre-analytical, post analytical and analytical workflow(s)/ workflow step(s). The control unit may receive information from a data management unit regarding which steps need to be performed with a certain sample. In some embodiments, the control unit might be integral with a data management unit, may be comprised by a server computer and/or be part of one instrument or even distributed across multiple instruments of the laboratory system. The control unit may, for instance, be embodied as a programmable logic controller running a computer-readable program provided with instructions to perform operations.

The term '**communication network**' as used herein encompasses any type of wireless network, such as a WIFI, GSM, UMTS or other wireless digital network or a cable based network, such as Ethernet or the like. In particular, the communication network can implement the Internet protocol (IP). For example, the communication network comprises a combination of cable-based and wireless networks. In embodiments wherein units of the system are comprised within one laboratory instrument, the communication network comprises communication channels within an instrument.

The term '**user interface**' as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

The term '**workflow**' as used herein encompasses any task that comprises a number of steps, such as for maintenance or operation of the system or one of its system components.

The term '**workflow step**' or '**processing step**' as used herein encompasses any activity belonging to a workflow. The activity can be of an elementary or complex nature and is typically performed at or by one or more instrument(s).

The terms '**sample**', '**patient sample**' and '**biological sample**' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

A **'STAT sample'** is a sample which needs to be processed and analyzed very urgently as the analysis result may be of life-crucial importance for a patient.

The term **'sample tube'** refers to any individual container for transporting, storing and/or processing a sample. In particular, said term without limitation refers to a piece of laboratory glass- or plastic-ware optionally comprising a cap on its upper end.

Sample tubes, e.g. sample tubes used to collect blood, often comprise additional substances such as clot activators or anticoagulant substances which have an impact on the processing of the sample. As a consequence, different tube types typically are adapted for pre-analytical and analytical requirements of a particular analysis, e.g. a clinical chemistry analysis, a hematological analysis or a coagulation analysis. A mix up of sample tube types can make (blood) samples unusable for analysis. To prevent errors in the collection and handling of samples, the sample caps of many tube manufacturers are encoded according to a fixed and uniform color scheme. Some sample tubes types in addition or alternatively are characterized by particular tube dimensions, cap dimensions, and/or tube color. A dimension of a tube comprises e.g. its height, its size and/or further characteristic shape properties.

The term **'sample rack'** is a carrier, typically made of plastics and/or metal, adapted for receiving, holding and transporting one or more sample tubes, e.g. 5 or more sample tubes, e.g. disposed in one or more rows. Apertures, windows or slits may be present to enable visual or optical inspection or reading of the sample tubes or of the samples in the sample tubes or of a label, such as a barcode, present on the sample tubes held in the sample rack.

The term **'tube type'** as used herein refers to a category of sample tubes which can be characterized by at least one shared property, whereby said shared property can be automatically detected by a lab-device and can thus be used to discriminate a set of sample tubes of a first tube type from another. Some tube types are designed for carrying samples which can be used for a plurality of different analytical tests. An example for such a tube type is a serum tube. However, a tube type may also be particular for one single analytical test.

The term **'sample input station'** as used herein refers to a part of a instrument or to an entire instrument configured to receive sample tubes before these are processed by the same instrument or transferred (by a transport system or manually) to another instrument of the laboratory system. The sample tubes may be loaded into the sample input station individually or rackwise.

The workcells may be connected by a transport system (conveyor and/or robotic arm). Alternatively, samples are transported from one workcell to the other manually or workcells are directly connected to each other.

The term **'analyte'** as used herein refers to a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term **'analysis'** or **'analytical test'** as used herein encompasses a laboratory procedure characterizing a parameter of a biological sample, e.g. light absorption, fluorescence, electrical potential or other physical or chemical characteristics of the reaction to provide the measurement data.

The term **'test order'** as used herein refers to any data object, computer loadable data structure, modulated data representing such data being indicative of one or more analytical tests to be executed on a particular biological sample. For example, a test order may be a file or an entry in a database. A test order can indicate an analytical test if, for example, the test order comprises or is stored in association with an identifier of an analytical test to be executed on a particular sample.

The term **'analytical data'** as used herein encompasses any data that is descriptive of a result of a measurement of a biological sample. In case of a calibration the analytical data comprises the calibration result, i.e. calibration data. In particular, the analytical data comprises an identifier of the sample for which the analysis has been performed and data being descriptive of a result of the analysis, such as measurement data.

The terms **'sorting'** and **'grouping'** will in the following be used synonymously in order to refer to the grouping of biological samples based on features shared by all samples of a particular group for processing all samples of a group in the same manner at least during a subsequent processing step.

Particular embodiments of the disclosed method/ system shall be described as follows with reference to the figures.

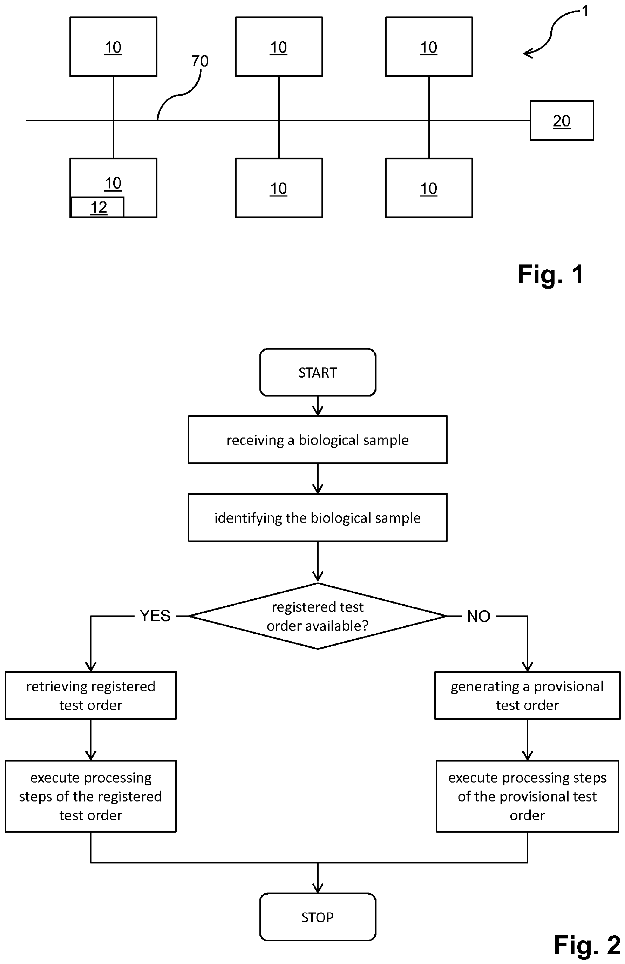

As shown on the block diagram of figure 1, embodiments of the disclosed laboratory system 1 for processing biological sample(s) comprise one or more laboratory instrument(s) 10, a sample input station 12 and a control unit 20 communicatively connected by a communication network 70.

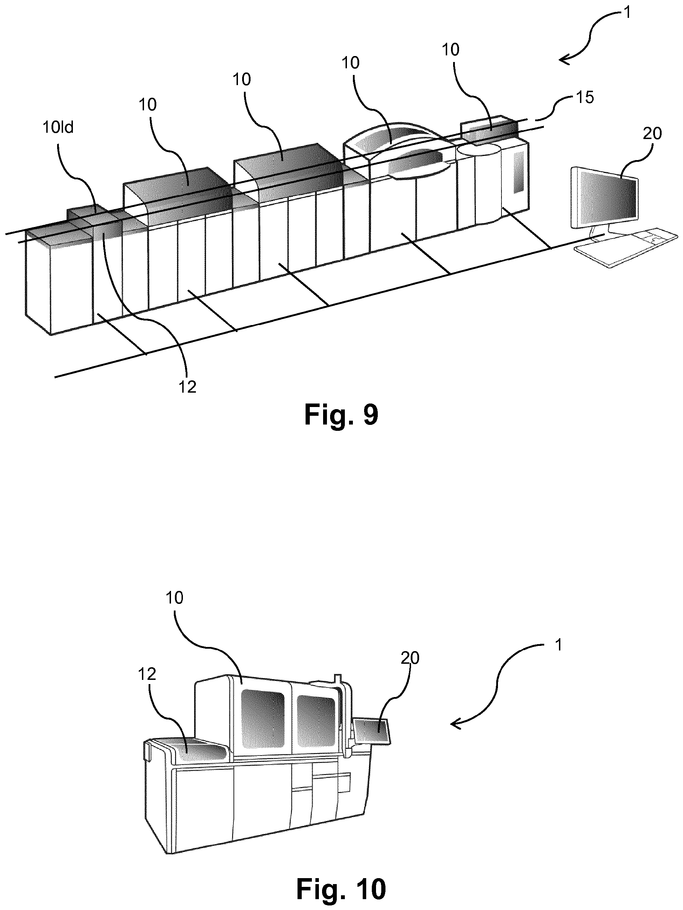

The one or more laboratory instrument(s) 10 are configured to execute processing steps on the biological samples according to instructions from the control unit 20. The one or more laboratory instrument(s) 10 may be one or more instruments from the list comprising pre-analytical instruments, post-analytical instruments and also analytical instruments. According to various embodiments of the disclosed laboratory system 1, the one or more laboratory instruments 10 may be identical or different instruments such as clinical- & immunochemistry analyzers, coagulation chemistry analyzers, immunochemistry analyzers, urine analyzers, nucleic acid analyzers, etc.. According to further embodiments, the sample input station 12 and the one or more laboratory instruments 10 are connected by a transportation system 15 (see figure 9)-such as a conveyor belt transportation system or a multi-dimensional sample tube transportation table- configured to transport sample tubes between the sample input station 12 and the one or more laboratory instruments 10.

The sample input station 12 is configured to receive biological samples comprised in sample tubes. The sample tubes may be loaded individually or in sample tube racks, each rack capable of holding one or more sample tubes.

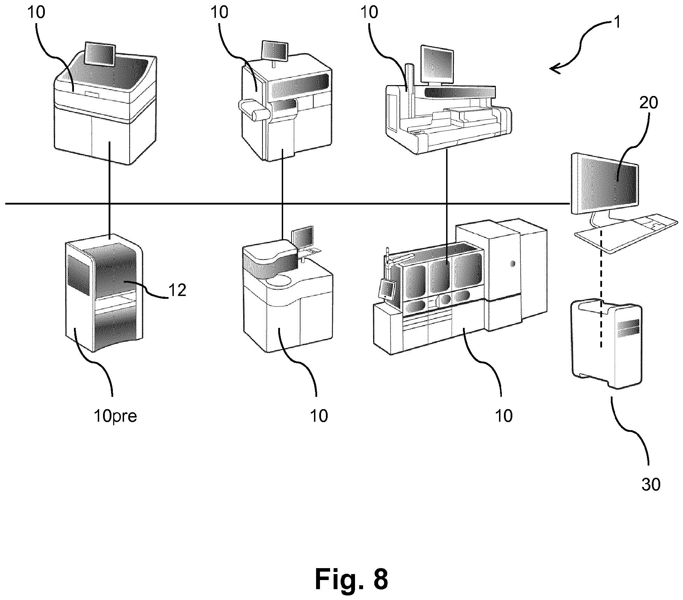

As it shall be described in greater detail with reference to figures 8 through 10, according to various embodiments of the disclosed laboratory system 1, the sample input station 12 is comprised within a pre-analytical laboratory instrument 10pre, comprised within one or more laboratory instruments 10 or it is a dedicated laboratory instrument for loading samples 10ld. The sample input station 12 comprises an identifier reader configured to identify the biological samples based on sample tube identifier(s) associated with the sample tube(s). According to embodiments of the disclosed system, the sample tube identifier is a bar code or an RFID tag. Correspondingly, the identifier reader is a barcode reader or an RFID reader.

A first embodiment of the method for operating a laboratory system 1 shall be now described as illustrated on the flowchart of figure 2.

As a first step, the sample input station 12 receives the biological sample, the biological sample being contained in a sample tube having a sample tube identifier.

After or upon receiving the sample tube, the sample input station 12 determines a sample ID of the biological sample based on the sample tube identifier of the sample tube.

Then the sample input station 12 transmits the sample ID of the biological sample to the control unit 20.

Upon receipt of the sample ID, the control unit 20 performs a check (determines) whether a test order has been registered and a definite test order 100 is available to the laboratory system 1 corresponding to the sample ID of the biological sample received, the definite test order 100 being indicative of one or more processing step(s) to be executed on the biological sample. The term **'definite'** with regard to a definite test order 100 refers to a test order which is registered/ entered for the laboratory system (directly or indirectly via a host system such as a laboratory system LIS), the definite test order 100 reflecting an actual order for the corresponding biological sample(s). Definite test orders 100 typically originate from a physician, a nurse, a clinical laboratory personnel etc. who requests (orders) one or more analytical test(s) be performed on particular biological sample. These one or more analytical test(s) ordered are registered into the system and translated into the one or more processing steps of the definite test order 100. The term '**is available'** - with respect to a test order being available to the laboratory system 1 - shall mean that a test order accessible to the laboratory system 1, either directly or indirectly via a communication network 70, irrespective whether the test order is stored in a local or remote database, a look-up table or other suitable means to store a test order.

Depending on the embodiment, the definite test order 100 for the at least one biological sample can be received based on a push- or a pull approach. For example, a test order 100 received via the push approach may be submitted to the laboratory system 1 from the LIS or another piece of laboratory software as soon as said test order is specified (entered by a user) for a particular biological sample. In the following, the expression 'receiving a test order' and ' a test order is available' encompasses any push- or pull-based approach for receiving a test order for a biological sample.

According to some pull-approach based embodiments, the control unit 20 attempts to pull (retrieve) a definite test order 100 for the at least one biological sample by executing a request. Depending on the embodiment, executing the request may comprise submitting an electronic request for a test order via a network 70 to the middleware or the LIS, e.g. a Web-service request, a remote procedure call or the like. Said request may also comprise executing a read operation on a computer readable storage medium in order to determine whether a definite test order 100 for said received biological sample has been stored to said storage medium.

If a corresponding definite test order 100 is available to the laboratory system 1, then the control unit 20 retrieves the corresponding definite test order 100 and instructs the laboratory system 1 to execute the one or more processing steps on the biological sample.

If a definite test order 100 corresponding to the biological sample received is not available (or not yet available) to the laboratory system 1, then a provisional test order 101 corresponding to the sample ID of the biological sample received is generated, the provisional test order 101 being indicative of one or more provisional processing steps to be executed on the biological sample. The term 'provisional' with regard to the provisional test order 101, respectively the provisional processing steps refers to the fact that provisional test orders are generated to (provisionally) bridge the time span from receipt of the biological sample by the sample input unit 12 until the definite test order 100 becomes available. In other words the definite test order 100 takes over from the provisional test order 101 (which is generated as compared to being registered). Nevertheless, the term 'provisional' shall not be interpreted to exclude cases when the provisional test order 101 defines all processing steps to be executed on the biological sample. Thus, according to embodiments, the provisional test order 101 may take over the role of the definite test order 100 if all processing steps required to be carried out on the biological sample can be automatically determined by the laboratory system 1, without the need for a definite test order 100 to be recorded.

According to embodiments, the provisional test order 101 is generated by the control unit 20. According to further embodiments the control unit 20 does by itself not generate but requests the test order from a host system 30, such as a laboratory information system. Upon receipt of such a request, the host system 30 generates the provisional test order 101 and transmits it to the control unit 20.

Once the provisional test order 101 has been generated, the control unit 20 instructs the laboratory system 1 to execute the one or more provisional processing steps on the one or more biological sample according to the provisional test order 101.

The expression 'being indicative' (with respect to a test order being indicative of one or more processing step(s) to be executed on the biological sample) implies that the test order itself may comprise instructions, e.g. computer interpretable instructions for executing the one or more processing steps on the biological sample. In addition or alternatively, the test order may comprise one or more identifiers of one or more analytical tests and/or pre-analytical and/or post-analytical processing steps to be executed on a sample. A test order may also be indicative of a complex sample processing workflow covering pre-analytical, analytical and/or post-analytical sample processing steps. Detailed computer interpretable instructions for triggering one or more laboratory instruments to execute the physical processing steps on the biological sample can be a part of the test order itself or can be stored elsewhere in association with one or more identifiers contained in said test order.

According to embodiments of the disclosed method/ system, the step of generating the provisional test order 101 comprises the steps of matching a template (mask or pattern) over the sample tube identifier and retrieving one or more processing steps to be carried out on biological samples identified by a sample tube identifier matching the template.

According to a particular embodiment, matching a template over the sample tube identifier involves identifying certain characters or character strings/ combinations in the sample tube identifier in order to determine the analytical test (processing steps) to be performed on the biological sample contained in that sample tube. For example a template for matching over sample tube identifiers has the following format:

xxx.xxx.**KKTT**

- where x represents any character (usually a serial number or the like)
- KK is one or two character code identifying a class of test, such as:

| | |
|---|---|
| Anemia | AN |
| Bone | B |
| Cardiac | CA |
| Coagulation | C |
| DAT | D |
| Endocriniology | E |
| Fertility | F |
| Hepatology | H |
| Infectious Diseases | ID |
| Inflammation | I |
| Metabolic | M |
| Oncology | O |
| Renal | R |
| TDM | TD |
| Women's Health | WH |
| Urine | U |

- T is a one or two character code identifying a test of the identified class, with specific processing steps to be executed on the biological sample,

Examples of sample identifiers and how the template above is applied:
123.456.**AN01** for test 01 (for example a Folate test) of the Anemia test class; or
123.457.**U12** for a Fibrinogen Coagulation test.

Once a template is matched, the one or more corresponding processing steps are retrieved and assigned to the provisional test order 101 to be carried out on the biological sample contained in the sample tube having the sample tube identifier matching the particular template.

Alternatively or additionally, according to embodiments of the disclosed method/ system, the step of generating the provisional test order 101 comprises the steps of identifying an interval to which the sample tube identifier belongs and retrieving of one or more processing steps to be carried out on biological samples identified by a sample tube identifier within the identified interval. Accordingly set intervals (ranges) of sample tube identifiers are associated with a particular analytical test (with corresponding processing steps) and/or class of analytical tests.

For example sample tube identifiers in the interval:

| | |
|---|---|
| 123.000 - 123.999 | are associated with analytical test 1; |
| 124.000 - 124.999 | are associated with analytical test 2; |
| etc. | |

Alternatively or additionally, according to embodiments of the disclosed method/ system, the step of generating the provisional test order 101 comprises the steps of determining the tube type of the sample tube that contains the biological sample and retrieving one or more processing steps to be carried out on biological samples received in samples tubes of the determined tube type.

According to embodiments, the tube type is determined automatically by the sample input station 12, e.g. by an image detection device being part of the input station 12. Depending on the embodiment of the invention, the determination of the tube type can be based on an analysis of one or more of the following features being selected from the group comprising:
- the color of the tube,
- the color of the tube cap,
- the dimensions of the tube (i.e. length and/or diameter and/or shape property),
- the dimensions of the tube cap (length and/or diameter and/or shape property),
- a tube type label being indicative of the tube type, e.g. a 2D or 3D code, e.g. a barcode or a matrix code.

A shape property can be, for example, depressions or elevations of the surface.

According to embodiments of the disclosed method/ system, the steps of matching a template over the sample tube identifier and/or by identifying an interval to which the sample tube identifier belongs and/or by determining the tube type are performed by the control unit 20 alone and/or by querying a database and/or a Laboratory Information System LIS.

In certain cases, by matching a template over the sample tube identifier and/or by identifying an interval to which the sample tube identifier belongs and/or by determining the tube type, only the test class can be determined but not the specific analytical test. In this case, the provisional test order 101 comprises pre-analytical sample processing steps which are common for all analytical tests of the identified test class, processing steps which prepare the sample for a succeeding analysis as far as possible based on the information provided by the analytical test class. For example in case based on the template and/or interval and/or tube type, the sample has been identified as a sample for a coagulation test, a centrifugation program is specified accordingly and the biological sample is centrifuged according to said program also in the absence of a definite test order. The provisional processing steps in this example are pre-analytical sample processing steps which prepare the sample for a succeeding coagulation analysis as far as possible based on the information provided by matching a template over the sample tube identifier and/or by identifying an interval to which the sample tube identifier belongs and/or by determining the tube type. For example, the provisional processing steps of the provisional test order 101 comprise: programming a centrifuge for executing a centrifugation step of 3000 g for 10 minutes; executing said centrifugation step on the sample; the sample is then forwarded to a buffering station. In the buffering station, the sample is stored until a definite test order 100 for the coagulation sample is available which is used to determine and execute processing steps specific for that particular analytical test.

Alternatively or additionally according to certain embodiments, if by matching a template over the sample tube identifier and/or by identifying an interval to which the sample tube identifier belongs and/or by determining the tube type, the specific analytical test to be carried out on the biological sample cannot be automatically determined, the control unit determines one or more most common analytical test(s) executed by the laboratory system 1 and takes over the corresponding processing steps to the provisional test order 101. The most common analytical test(s) are determined based on historical statistical data and therefore reflect the analytical test(s) which is/are more likely to be ordered (in the definite test order 100) for the newly received biological sample. Such embodiments are advantageous in use scenarios where the urgency of obtaining the results of the analytical test are of very high importance, such as in an emergency care unit.

According to embodiments of the disclosed method/ system, the provisional processing steps of the provisional test order 101 are executed not on the primary sample but on an aliquot of the biological sample and wherein all the processing steps of the definite test order 100 are carried out on the biological sample if one or more of the executed provisional processing steps of the provisional test order 101 executed by the laboratory system 1 on the biological sample conflicts with one or more processing steps of the definite test order 100. Such embodiments are advantageous especially if certain assumptions have been made in the process of generating the provisional test order 101 which might have led to provisional processing steps being executed on the biological sample which are in conflict with the definite test order 100. By executing the provisional processing steps on aliquots and not the primary biological sample, the processing steps of the definite test order 100 may still be carried out successfully despite a conflict of the provisional test order 101. According to embodiments, the control unit 20 instructs the laboratory system 1 to carry out the provisional processing steps of the provisional test order 101 on an aliquot of the biological sample only if a quantity (volume) of the biological sample is sufficient so that even after aliquoting, sufficient (primary) sample volume left is available for further analytical test(s) in case the provisional processing steps of the provisional test order 101 conflict with one or more processing steps of the definite test order 100 rendering the aliquot unsuitable for the processing steps of the definite test order 100. In other words a certain quantity (volume) of biological sample is kept as a backup in case the generated test order 101 is erroneous.

According to some embodiments, the one or more provisional processing steps of the provisional test order comprise one final step of transferring the at least one biological sample to a storage unit after having executed all provisional processing steps. According to some of said embodiments, said final transportation step is not executed if, while executing one of the provisional processing steps of the provisional test order, a definite test order was received. This is advantageous, because it avoids executing unnecessary transportation steps (to and from a sample storage unit).

According to embodiments, the laboratory system 1 comprises at least one centrifuge. In case a definite test order 100 is available, the one or more first processing steps thereof comprise at least one centrifugation step, whereby said centrifugation step is executed by said at least one centrifuge as indicated by the definite test order 100. In case a definite test order 100 is not available, the one or more second processing steps of the provisional test order 101 may also comprise at least one centrifugation step(s). In this case, said centrifugation step is determined in dependence on the provisional test order 101 based on a determined test class. This is advantageous, because centrifugation steps can take a considerable amount of time and are often the time limiting step of a workflow. Therefore, executing a centrifugation step based on a provisional test order 101 in case a definite test order 100 is not available helps to avoid bottlenecks. In many cases, delays resulting from a delayed registration (e.g. by lab personnel) of a definite test order 100 to a sample can be completely avoided, because the centrifugation step can be started based on the provisional test order 101. As a centrifugation step may take 5-20 mins., there is a good chance the definite test order 100 becomes available by the time the provisional processing step of centrifugation is completed.

According to further embodiments, the laboratory system 1 comprises at least one aliquotation station.

According to further embodiments, the laboratory system 1 comprises at least one decapping and/or recapping station. In case a definite test order is available, the one or more processing steps of the definite test order 100 comprise at least one decapping and/or recapping step to be executed on said at least one biological sample by the at least one decapping and/or recapping station. In case a definite test order 100 was not received, the one or more provisional processing steps of the provisional test order 101 may comprise said at least one decapping and/or recapping step. This is advantageous, because capping, decapping and recapping a sample may be a necessary pre-analytical procedure in a variety of different workflow settings.

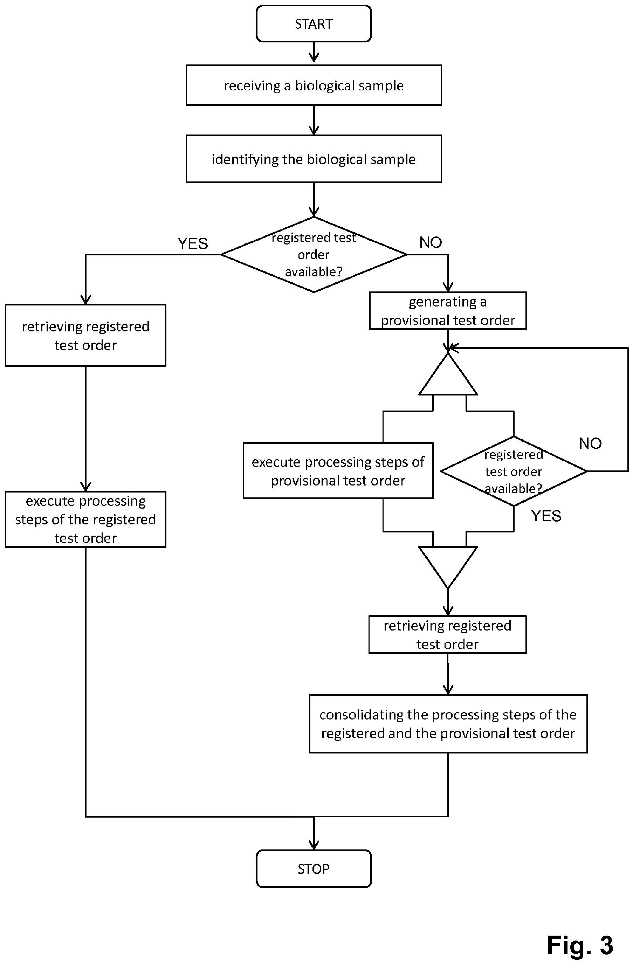

Figure 3 shows a flowchart illustrating a further embodiment of the disclosed method for operating a laboratory system 1, wherein after instructing the laboratory system 1 to execute the one or more processing steps according to the provisional test order 101, the control unit 20 periodically determines whether a definite test order 100 corresponding to the biological sample has become available to the laboratory system 1. The step of periodically determining whether a definite test order 100 has become available may be implemented depending on the embodiment as a push- or a pull approach, that is a definite test order 100 may be pushed to the laboratory system 1 by a host system 30 or in a pull approach, the control unit 20 attempts to pull (retrieve) a definite test order 100 for the at least one biological sample by executing a request. Typically the definite test order 100 becomes available shortly after an the order has been registered / entered by an operator. However delays might occur due to transmission interruptions, slow connection or validation steps required before an order registered is marked as a definite test order 100.

As illustrated on figure 3, if a definite test order 100 corresponding to the biological sample did become available to the laboratory system 1, the control unit 20 retrieves the definite test order 100 and consolidates the processing step(s) of the definite test order 100 and the provisional processing step(s) of the provisional test order 101.

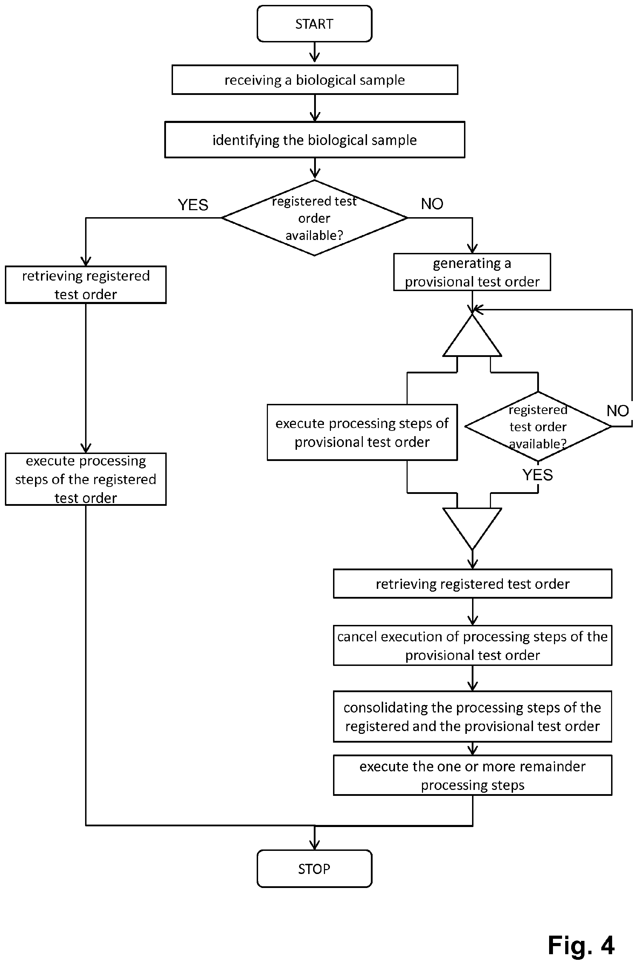

Figure 4 shows a flowchart illustrating an even further embodiment of the disclosed method for operating a laboratory system 1, wherein the step of consolidating the processing step(s) of the definite test order 100 and the provisional test order 101 comprises determining one or more executed provisional processing steps of the provisional test order 101 which have already been executed by the laboratory system 1. Processing steps already started and which cannot be cancelled without affecting the biological sample are considered in this respect as executed processing steps. Thereafter one or more remainder processing steps of the definite test order 100 with no corresponding executed provisional processing steps are identified. In this context, processing steps are considered as corresponding processing steps, when their execution results in the same (or analytically the same) end effect on the biological sample. Otherwise the provisional processing steps of the provisional test order 101 are said to be in conflict with the remainder processing steps of the definite test order 100.

For example a provisional processing step of the provisional test order 101 indicates that the biological sample needs to centrifuged at a first RPM for a first period and the definite test order 100 indicates that the biological sample needs to centrifuged at a second RPM for second period. If the end centrifuging effect on the biological sample is the same or analytically the same, that is the centrifugation according to the provisional test order is also suitable for the analytical test to be carried out according to the definite test order 100, then the provisional processing step is considered to correspond to the processing step of the definite test order 100. However, if the centrifugation according to the provisional test order is not suitable for the analytical test to be carried out according to the definite test order 100, then the provisional processing step is considered to conflict with the processing step of the definite test order 100. Furthermore if for example a processing step of the provisional test order 101 instructs a different laboratory instrument 10 to process the biological sample than the corresponding definite test order 100, but the analytical test is actually the same (or analytically the same), then the processing step of the provisional test order 101 is said to correspond to (and not conflict with) the processing step of the definite test order 100 despite the fact that it was carried out by a different instrument. Otherwise (no conflict) there is no need to cancel the execution of the provisional processing steps.

As shown on figure 4, once the executed provisional processing steps of the provisional test order 101 as well as the remainder processing steps of the definite test order 100 have been determined, the control unit 20 instructs the laboratory system 1 to cancel execution of provisional processing steps of the provisional test order 101 not yet executed and to execute the one or more remainder processing steps of the definite test order 100 on the biological sample, if the provisional processing steps of the provisional test order 101 not yet executed conflict with the remainder processing steps of the definite test order 100.

According to further embodiments, if all the provisional processing steps of the provisional test order 101 correspond to the processing steps of the definite test order 100, then the analytical data (i.e. the result(s)) obtained during the execution of the one or more provisional processing steps are assigned to the definite test order 100.

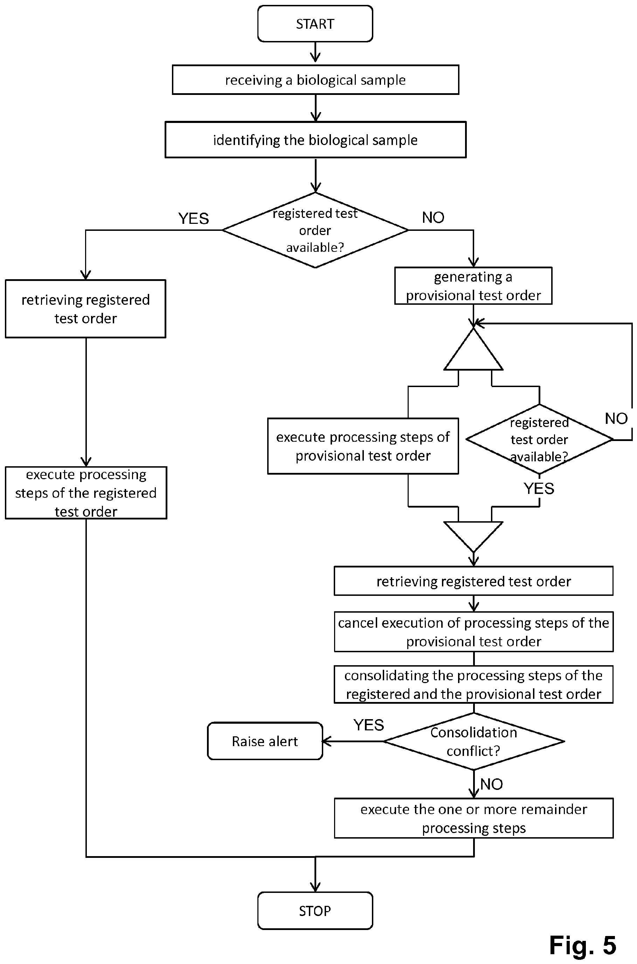

Figure 5 shows a flowchart illustrating an even further embodiment of the disclosed method for operating a laboratory system 1, wherein an alert is raised if one or more of the executed provisional processing steps of the provisional test order 101 executed by the laboratory system 1 on the biological sample conflicts with one or more processing steps of the definite test order 100. The alert may be in the form of a visual, audible or tactile alert displayed and/or emitted from the control unit 20 and/or one or more laboratory instrument 10, respectively on a user interface thereof. Alternatively or additionally, the alert is transmitted via the communication network 70 to the host system 30. It is then up to the operator or the host system 30 to handle the alert. For example by generating a new test order for a new sample from the same patient or a different aliquot of the same sample if one is available.

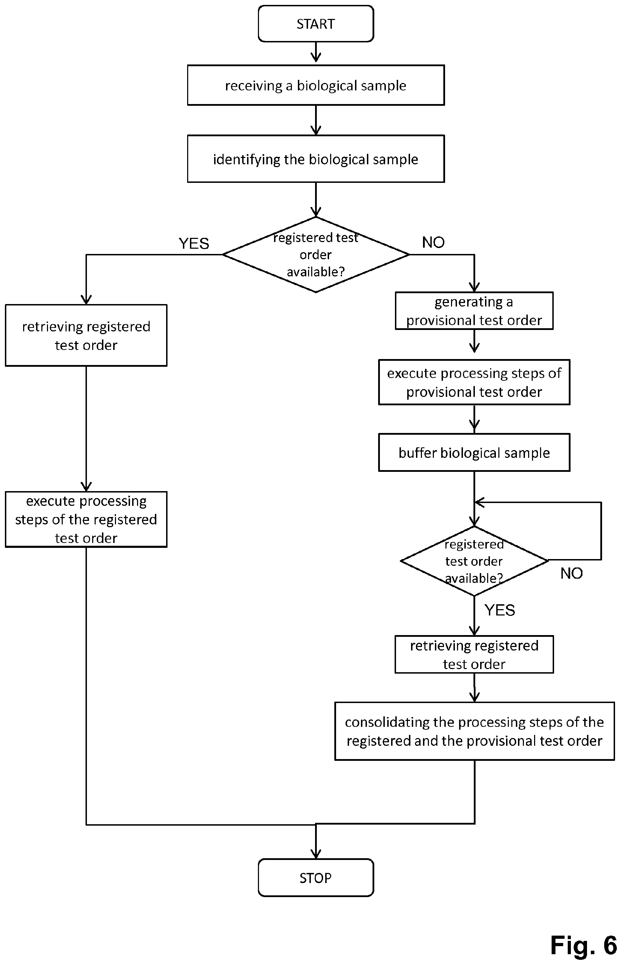

Figure 6 shows a flowchart illustrating an even further embodiment of the disclosed method for operating a laboratory system 1, wherein the control unit 20 instructs the laboratory instrument 1 to buffer the biological sample after execution of the one or more provisional processing steps of the provisional test order 101. In this context the term 'buffer' refers to the process of temporally storing the biological sample, in particular in a temperature controlled environment.

According to embodiments of the disclosed method/ system, the biological sample is buffered in a central buffer unit of the laboratory system, the central buffer unit being in common for a plurality of laboratory instruments 10 and configured to temporarily store sample tubes.

According to particular embodiments, the central buffer unit is coupled to the laboratory system 1 by a sample tube transport system, such as a conveyor belt transportation system or a multi-dimensional sample tube transportation table- configured to transport sample tubes between the sample input station 12; the one or more laboratory instruments 10 and the central buffer unit.

According to particular embodiments, biological samples are flagged/ marked to be processed with priority once a corresponding definite test order 100 becomes available so as to avoid the biological sample to remain buffered for longer than necessary. Alternatively or additionally, the biological samples may be buffered to a sample archive. The control unit 20 is further configured to retrieve the biological sample (from the buffer) once the definite test order (100) becomes available. Such embodiment are advantageous as provisional processing steps may be carried out on the biological sample (while a definite test order is not available) and then temporarily buffered until the analytical test can be carried out once the definite test order becomes available. Hence accumulation of sample tubes at the sample input station 12 can be prevented.

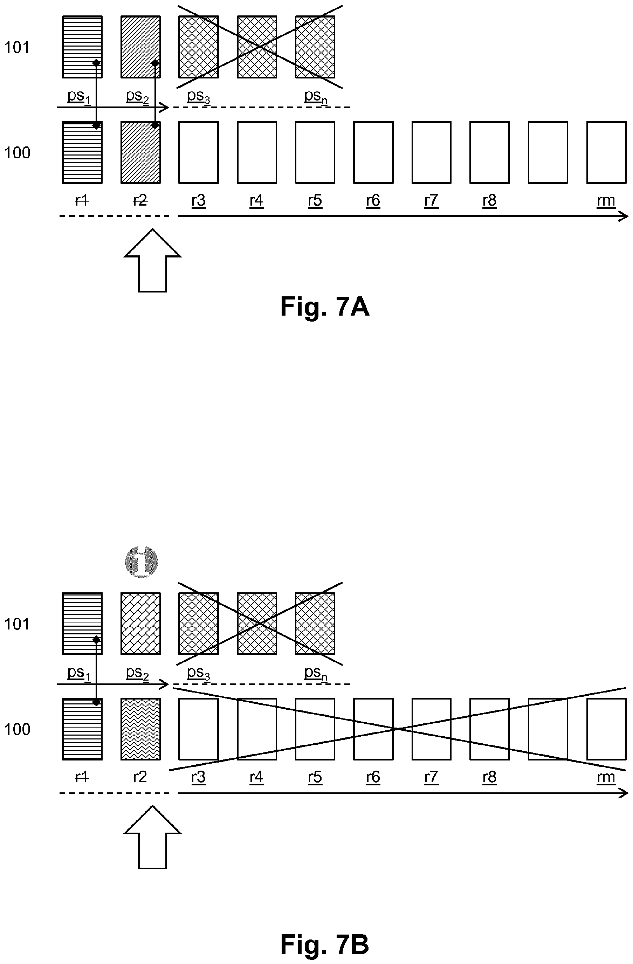

Figure 7A shows an illustration of execution of processing steps, illustrating the cancelation of the execution of provisional processing steps ps₃ through psₙ of a provisional test order 101 and handover to the execution of remainder processing steps r₃ through rₘ of a definite test order 100, the availability of the definite test order 100 being shown with a block arrow. As illustrated, the already executed (at the time the definite test order 100 becomes available) provisional processing steps ps₁ and ps₂ both have a corresponding processing step r1 respectively r₂ of the definite test order 100. Hence there is no conflict between the executed provisional processing steps and the processing steps of the definite test order 100. Therefore the control unit 20 instructs the laboratory system 1 to execute the one or more remainder processing steps r₃ through rₘ of the definite test order 100.

Figure 7B on the other hand illustrates a case when - at the moment the definite test order 100 becomes available- the laboratory system 1 already executed a provisional processing step ps₃ which conflicts with the remainder processing steps r₂ through rₘ of the definite test order 100. Hence an alert is raised and the operation must be cancelled.

Figure 8 shows a schematic illustration of an embodiment of the disclosed laboratory system 1 wherein the sample input station 12 is comprised within a pre-analytical laboratory instrument 10pre.

Figure 9 shows a schematic illustration of an embodiment of the disclosed laboratory system 1 wherein the sample input station 12 is a dedicated laboratory instrument 10ld for loading samples and where the sample input station 12 and the one or more laboratory instruments 10 are connected by a sample transportation system 15 such as a conveyor belt transportation system configured to transport sample tubes between the sample input station 12 and the one or more laboratory instruments 10.

Figure 10 shows a schematic illustration of an embodiment of the disclosed laboratory system 1 integrated into a single laboratory instrument.

Further disclosed and proposed is a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps as disclosed herein may be performed by using a computer or a computer network, preferably by using a computer program.

Further disclosed and proposed is a computer program product having program code means, in order to perform the method according to the present invention in one or more of the embodiments enclosed herein when the program is executed on a computer or computer network. Specifically, the program code means may be stored on a computer-readable data carrier.

Further disclosed and proposed is a data carrier having a data structure stored thereon, which, after loading into a computer or computer network, such as into a working memory or main memory of the computer or computer network, may execute the method according to one or more of the embodiments disclosed herein.

Further disclosed and proposed is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Further disclosed and proposed is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing measurements.

Further disclosed and proposed is a computer or computer network comprising at least one processor, wherein the processor is adapted to perform the method according to one of the embodiments described in this description.

Further disclosed and proposed is a computer loadable data structure that is adapted to perform the method according to one of the embodiments described in this description while the data structure is being executed on a computer,

Further disclosed and proposed is a storage medium, wherein a data structure is stored on the storage medium and wherein the data structure is adapted to perform the method according to one of the embodiments described in this description after having been loaded into a main and/or working storage of a computer or of a computer network.

**REFERENCE LIST:**

| | |
|---|---|
| laboratory system | 1 |
| laboratory instrument | 10 |
| pre-analytical laboratory instrument | 10pre |
| laboratory instrument for loading samples | 10ld |
| sample input station | 12 |
| sample transportation system | 15 |
| control unit | 20 |
| host system | 30 |
| communication network | 70 |
| definite test order | 100 |
| provisional test order | 101 |

## Claims

1. A method for operating a laboratory system (1) comprising one or more laboratory instrument(s) (10), a sample input station (12) and a control unit (20) communicatively connected by a communication network (70), the method comprising the steps of:
- receiving a biological sample by the sample input station (12), the biological sample being contained in a sample tube having a sample tube identifier;
- the sample input station (12) determining a sample ID of the biological sample by reading the sample tube identifier of the sample tube;
- the sample input station (12) transmitting the sample ID of the biological sample to the control unit (20);
- the control unit (20) determining whether a definite test order (100) is available to the laboratory system (1) corresponding to the sample ID of the biological sample received, the definite test order (100) being indicative of one or more processing step(s) to be executed on the biological sample;
- if a definite test order (100) corresponding to the biological sample received is available to the laboratory system (1):
- the control unit (20) retrieving the corresponding definite test order (100) and instructing the laboratory system (1) to execute the one or more processing steps on the biological sample;
- if a definite test order (100) corresponding to the biological sample received is not available to the laboratory system (1):
- generating a provisional test order (101) corresponding to the sample ID of the biological sample received, the provisional test order (101) being indicative of one or more provisional processing steps to be executed on the biological sample; and
- the control unit (20) instructing the laboratory system (1) to execute the one or more provisional processing steps on the one or more biological sample according to the provisional test order (101);
**characterized in that**
- if a definite test order (100) corresponding to the biological sample received is not available to the laboratory system (1) and if a provisional test order (101) corresponding to the sample ID cannot be automatically generated, the step of generating the provisional test order (101) comprises the steps of:
- determining one or more most common analytical test(s) executed by the laboratory system;
- retrieving one or more processing steps corresponding to the most common analytical test(s).

2. A method for operating a laboratory system (1) according to claim 1, further comprising the steps of:
- after instructing the laboratory system (1) to execute the one or more provisional processing steps, the control unit (20) periodically determining whether a definite test order (100) corresponding to the biological sample has become available to the laboratory system (1);
- if a definite test order (100) corresponding to the biological sample has become available to the laboratory system (1), the control unit (20) retrieving the definite test order (100); and
- the control unit (20) consolidating the processing step(s) of the definite test order (100) and the provisional processing step(s) of the provisional test order (101)
if a definite test order (100) corresponding to the biological sample was not available upon receiving the biological sample by the sample input station (12).

3. A method for operating a laboratory system (1) according to claim 2, wherein the step of consolidating the processing step(s) of the definite test order (100) and the provisional test order (101) comprises:
- determining one or more executed provisional processing steps of the provisional test order (101) which have already been executed by the laboratory system (1);
- determining one or more remainder processing steps of the definite test order (100) with no corresponding executed provisional processing steps,
the method further comprising the steps of:
- if the provisional processing steps of the provisional test order (101) not yet executed conflict with the remainder processing steps of the definite test order (100), the control unit (20) instructing the laboratory system (1) to cancel execution of provisional processing steps of the provisional test order (101) not yet executed and to execute the one or more remainder processing steps of the definite test order (100) on the biological sample.

4. A method for operating a laboratory system (1) according to one of the preceding claims, wherein the control unit (20) instructs the laboratory instrument (10) to buffer the biological sample after execution of the one or more provisional processing steps of the provisional test order (101) and to retrieve the biological sample once the definite test order (100) becomes available.

5. A method for operating a laboratory system (1) according to claim 3 or 4, further comprising the step of raising an alert if one or more of the executed provisional processing steps of the provisional test order (101) executed by the laboratory system (1) on the biological sample conflicts with one or more processing steps of the definite test order (100).

6. A method for operating a laboratory system (1) according to one of the preceding claims, wherein the step of generating the provisional test order (101) comprises the steps of:
- matching a template over the sample tube identifier; and
- retrieving one or more processing steps to be carried out on biological samples identified by a sample tube identifier matching the template.

7. A method for operating a laboratory system (1) according to one of the preceding claims,, wherein the step of generating the provisional test order (101) comprises the steps of:
- identifying an interval to which the sample tube identifier belongs; and
- retrieving one or more processing steps to be carried out on biological samples identified by a sample tube identifier within the identified interval.

8. A method for operating a laboratory system (1) according to one of the preceding claims, wherein the step of generating the provisional test order (101) comprises the steps of:
- determining the tube type of the sample tube that contains said one or more biological sample; and
- retrieving one or more processing steps to be carried out on biological samples received in samples tubes of the determined tube type.

9. A method for operating a laboratory system (1) according to one of the preceding claims, further comprising one or more of the steps:
- the control unit (20) requesting a test order (100, 101) from a host system (30), such as a laboratory information system;
- the host system (30) generating the provisional test order (101);
- the host system (30) transmitting the provisional test order (101) to the control unit (20);
- the host system (30) receiving the definite test order (101) from an operator via a user interface;
- the host system (30) transmitting the definite test order (100) to the control unit (20).

10. A method for operating a laboratory system (1) according to one of the preceding claims, wherein the provisional processing steps of the provisional test order (101) are executed on an aliquot of the biological sample and wherein all the processing steps of the definite test order (100) are carried out on the biological sample if one or more of the executed provisional processing steps of the provisional test order (101) executed by the laboratory system (1) on the biological sample conflicts with one or more processing steps of the definite test order (100).

11. A laboratory system (1) for processing biological sample(s), the laboratory system (1) comprising:
- one or more laboratory instrument(s) (10) configured to execute processing steps on the biological sample,
- a sample input station (12) configured to receive a biological sample comprised in a sample tube, the sample input station (12) comprising an identifier reader configured to determine a sample ID of the biological sample by reading a sample tube identifier associated with the sample tube and further configured to transmit the sample ID of the biological sample to the control unit (20);
- a control unit (20) configured to:
- determine whether a definite test order (100) is available to the laboratory system (1) corresponding to the sample ID of the biological sample received, the definite test order (100) being indicative of one or more processing step(s) to be executed on the biological sample;
- if a definite test order (100) corresponding to the biological sample received is available to the laboratory system (1):
- retrieve the corresponding definite test order (100) and instruct the laboratory system (1) to execute the one or more processing steps on the biological sample;
- if a definite test order (100) corresponding to the biological sample received is not available to the laboratory system (1)
- generate a provisional test order (101) corresponding to the sample ID of the biological sample received, the provisional test order (101) being indicative of one or more provisional processing steps to be executed on the biological sample; and
- instruct the laboratory system (1) to execute the one or more provisional processing steps on the one or more biological sample according to the provisional test order (101);
**characterized in that**
- if a definite test order (100) corresponding to the biological sample received is not available to the laboratory system (1) and if a provisional test order (101) corresponding to the sample ID cannot be automatically generated, the step of generating the provisional test order (101) comprises the steps of:
- determining one or more most common analytical test(s) executed by the laboratory system;
- retrieving one or more processing steps corresponding to the most common analytical test(s).

12. A laboratory system (1) according to claim 11, wherein the control unit (20) is further configured to:
- periodically determine whether a definite test order (100) corresponding to the biological sample has become available to the laboratory system (1) and;
- to retrieve the definite test order (100) if a definite test order (100) corresponding to the biological sample has become available to the laboratory system (1) and to consolidate the processing step(s) of the definite test order (100) and the provisional processing step(s) of the provisional test order (101).

13. A laboratory system (1) according to claim 12, wherein the control unit (20) is further configured to:
- determine one or more executed provisional processing steps of the provisional test order (101) which have already been executed by the laboratory system (1);
- determine one or more remainder processing steps of the definite test order (100) with no corresponding executed provisional processing steps;
- instruct the laboratory system (1) to cancel execution of provisional processing steps of the provisional test order (101) not yet executed;
- instruct the laboratory system (1) to execute the one or more remainder processing steps of the definite test order (100) on the biological sample.

14. A laboratory system (1) according to claim 12 or 13, wherein the control unit (20) is further configured to generate an alert if one or more of the executed provisional processing steps of the provisional test order (101) executed by the laboratory system (1) on the biological sample conflicts with one or more processing steps of the definite test order (100).

15. A laboratory system (1) according to one of the claims 11 to 14, wherein the control unit (20) is further configured to generate a provisional test order by performing the steps of:
- matching a template over the sample tube identifier and retrieve the one or more processing steps to be carried out on the biological sample identified by sample tube identifier matching the template; and/or
- determining the tube type of the sample tube that contains the biological sample and retrieve the one or more processing steps to be carried out on biological samples received in samples tubes of the determined tube type; and/or
- identifying an interval to which the sample tube identifier belongs and retrieve the one or more processing steps to be carried out on biological samples identified by sample tube identifiers within the identified interval.

16. A laboratory system (1) according to one of the claims 11 to 15, wherein the sample input station (12) is comprised within a pre-analytical laboratory instrument (10pre), and/or the sample input station (12) is comprised within one or more laboratory instruments (10); and/or the sample input station (12) is a dedicated laboratory instrument for loading samples (10ld).

17. A laboratory system (1) according to one of the claims 11 to 15, wherein the sample input station (12) and the one or more laboratory instruments (10) are connected by a sample tube transportation system configured to transport sample tubes between the sample input station (12) and the one or more laboratory instruments (10).

## Patentansprüche

1. Verfahren zum Betreiben eines Laborsystems (1), umfassend einen oder mehrere Laborapparat(e) (10), eine Probenzufuhrstation (12) und eine Steuereinheit (20), die kommunizierend mit einem Kommunikationsnetzwerk (70) verbunden ist, wobei das Verfahren die Schritte umfasst:
- Aufnehmen einer biologischen Probe durch die Probenzufuhrstation (12), wobei die biologische Probe in einem Probenröhrchen mit einer Probenröhrchen-Kennzeichnung enthalten ist;
- die Probenzufuhrstation (12) bestimmt eine Proben-ID der biologischen Probe durch Lesen der Probenröhrchen-Kennzeichnung des Probenröhrchens;
- die Probenzufuhrstation (12) übermittelt die Proben-ID der biologischen Probe an die Steuereinheit (20);
- die Steuereinheit (20) bestimmt, ob dem Laborsystem (1) ein konkreter Prüfauftrag (100) vorliegt, der der Proben-ID der aufgenommenen biologischen Probe entspricht, wobei der konkrete Prüfauftrag (100) einen oder mehrere an der biologischen Probe auszuführende Verarbeitungsschritt(e) angibt;
- wenn dem Laborsystem ein konkreter Prüfauftrag (100) vorliegt, der der aufgenommenen biologischen Probe entspricht:
- ruft die Steuereinheit (20) den entsprechenden konkreten Prüfauftrag (100) ab und veranlasst das Laborsystem (1), den einen oder die mehreren Verarbeitungsschritte an der biologischen Probe auszuführen;
- wenn dem Laborsystem (1) kein konkreter Prüfauftrag (100) vorliegt, der der aufgenommen biologischen Probe entspricht:
- Erzeugen eines provisorischen Prüfauftrages (101), der der Proben-ID der aufgenommenen biologischen Probe entspricht, wobei der provisorische Prüfauftrag (101) einen oder mehrere an der biologischen Probe auszuführende provisorische Verarbeitungsschritte angibt; und
- veranlasst die Steuereinheit (20) das Laborsystem (1), den einen oder die mehreren provisorischen Verarbeitungsschritte an der einen oder den mehreren biologischen Proben gemäß dem provisorischen Prüfauftrag (101) auszuführen;
**dadurch gekennzeichnet, dass**
- wenn dem Laborsystem (1) kein konkreter Prüfauftrag (100) vorliegt, der der aufgenommenen biologischen Probe entspricht, und
- wenn nicht automatisch ein provisorischer Prüfauftrag (101) erzeugt werden kann, der der der Proben-ID entspricht, umfasst der Schritt des Erzeugens des provisorischen Prüfauftrages (101) die Schritte:
- Bestimmen einer oder mehrerer gängigster analytischer Untersuchung(en), die von dem Laborsystem ausgeführt werden;
- Abrufen eines oder mehrerer Verarbeitungsschritte, die den gängigsten analytischen Untersuchung(en) entsprechen.

2. Verfahren zum Betreiben eines Laborsystems (1) nach Anspruch 1, ferner umfassend die Schritte:
- nachdem das Laborsystem (1) veranlasst worden ist, den einen oder die mehreren provisorischen Verarbeitungsschritte auszuführen, bestimmt die Steuereinheit (20) periodisch, ob dem Laborsystem (1) ein konkreter Prüfauftrag (100), der der biologischen Probe entspricht, vorgelegt wurde;
- wenn dem Laborsystem (1) ein konkreter Prüfauftrag (100), der der biologischen Probe entspricht, vorgelegt wurde, ruft die Steuereinheit (20) den konkreten Prüfauftrag (100) ab; und
- die Steuereinheit (20) vereinigt die Verarbeitungsschritt(e) des konkreten Prüfauftrags (100) und die provisorischen Verarbeitungsschritt(e) des provisorischen Prüfauftrags (101),
wenn ein konkreter Prüfauftrag (100), der der biologischen Probe entspricht, nicht vorlag, nachdem die Probenzufuhrstation (12) die biologische Probe aufgenommen hat.

3. Verfahren zum Betreiben eines Laborsystems (1) nach Anspruch 2, wobei der Schritt des Vereinigens der Verarbeitungsschritt(e) des konkreten Prüfauftrags (100) und des provisorischen Prüfauftrags (101) umfasst:
- Bestimmen eines oder mehrerer ausgeführter provisorischer Verarbeitungsschritte des provisorischen Prüfauftrags (101), die bereits von dem Laborsystem (1) ausgeführt worden sind;
- Bestimmen eines oder mehrerer restlicher Verarbeitungsschritte des konkreten Prüfauftrags (100) ohne entsprechende ausgeführte provisorische Verarbeitungsschritte,
wobei das Verfahren ferner die folgenden Schritte umfasst:
- wenn die noch nicht ausgeführten provisorischen Verarbeitungsschritte des provisorischen Prüfauftrags (101) mit den restlichen Verarbeitungsschritten des konkreten Prüfauftrags (100) in Konflikt stehen, veranlasst die Steuereinheit (20) das Laborsystem (1), die Ausführung noch nicht ausgeführter provisorischer Verarbeitungsschritte des provisorischen Prüfauftrags (101) aufzuheben und den einen oder die mehreren restlichen Verarbeitungsschritte des konkreten Prüfauftrags (100) an der biologischen Probe auszuführen.

4. Verfahren zum Betreiben eines Laborsystems (1) nach einem der vorhergehenden Ansprüche, wobei die Steuereinheit (20) den Laborapparat (10) veranlasst, die biologische Probe nach der Ausführung des einen oder der mehreren provisorischen Verarbeitungsschritte des provisorischen Prüfauftrags (101) zu puffern und die biologische Probe abzurufen, sobald der konkrete Prüfauftrag (100) vorliegt.

5. Verfahren zum Betreiben eines Laborsystems (1) nach Anspruch 3 oder 4, ferner umfassend den Schritt des Auslösens eines Alarms, wenn einer oder mehrere der von dem Laborsystem (1) an der biologischen Probe ausgeführten provisorischen Verarbeitungsschritte des provisorischen Prüfauftrags (101) mit einem oder mehreren Verarbeitungsschritten des konkreten Prüfauftrags (100) in Konflikt stehen.

6. Verfahren zum Betreiben eines Laborsystems (1) nach einem der vorhergehenden Ansprüche, wobei der Schritt des Erzeugens des provisorischen Prüfauftrags (101) die Schritte umfasst:
- Abgleichen einer Vorlage mit der Probenröhrchen-Kennzeichnung und
- Abrufen eines oder mehrerer Verarbeitungsschritte, die an biologischen Proben durchzuführen sind, die mithilfe einer Probenröhrchen-Kennzeichnung, die der Vorlage gleicht, identifiziert worden sind.

7. Verfahren zum Betreiben eines Laborsystems (1) nach einem der vorhergehenden Ansprüche, wobei der Schritt des Erzeugens des provisorischen Prüfauftrags (101) die Schritte umfasst:
- Identifizieren eines Intervalls, zu dem die Probenröhrchen-Kennzeichnung gehört; und
- Abrufen eines oder mehrerer Verarbeitungsschritte, die an biologischen Proben durchzuführen sind, die mithilfe der Probenröhrchen-Kennzeichnung innerhalb des identifizierten Intervalls identifiziert worden sind.

8. Verfahren zum Betreiben eines Laborsystems (1) nach einem der vorhergehenden Ansprüche, wobei der Schritt des Erzeugens des provisorischen Prüfauftrags (101) die Schritte umfasst:
- Bestimmen der Röhrchenart des Probenröhrchens, das die eine oder die mehreren biologischen Proben enthält; und
- Abrufen eines oder mehrerer Verarbeitungsschritte, die an biologischen Proben, die in Probenröhrchen der bestimmten Röhrchenart aufgenommen sind, durchzuführen sind.

9. Verfahren zum Betreiben eines Laborsystems (1) nach einem der vorhergehenden Ansprüche, ferner umfassend einen oder mehrere der Schritte:
- die Steuereinheit (20) fragt einen Prüfauftrag (100, 101) von einem Host-System (30) wie einem Laborinformationssystem ab;
- das Host-System (30) erzeugt den provisorischen Prüfauftrag (101);
- das Host-System (30) übermittelt den provisorischen Prüfauftrag (101) an die Steuereinheit (20);
- das Host-System (30) empfängt den konkreten Prüfauftrag (101) von einem Bediener über eine Benutzerschnittstelle;
- das Host-System (30) übermittelt den konkreten Prüfauftrag (100) an die Steuereinheit (20).

10. Verfahren zum Betreiben eines Laborsystems (1) nach einem der vorhergehenden Ansprüche, wobei die provisorischen Verarbeitungsschritte des provisorischen Prüfauftrags (101) an einem Aliquot der biologischen Probe ausgeführt werden, und wobei alle Verarbeitungsschritte des konkreten Prüfauftrags (100) an der biologischen Probe durchgeführt werden, wenn einer oder mehrere der ausgeführten provisorischen Verarbeitungsschritte des provisorischen Prüfauftrags (101), die von dem Laborsystem (1) an der biologischen Probe ausgeführt werden, mit einem oder mehreren Verarbeitungsschritten des konkreten Prüfauftrags (100) in Konflikt stehen.

11. Laborsystem (1) zum Verarbeiten biologischer Probe(n), wobei das Laborsystem (1) umfasst:
- einen oder mehrere Laborapparat(e) (10), die so ausgebildet sind, dass sie Verarbeitungsschritte an der biologischen Probe ausführen,
- eine Probenzufuhrstation (12), die so ausgebildet ist, dass sie eine in einem Probenröhrchen enthaltene biologische Probe aufnimmt, wobei die Probenzufuhrstation (12) ein Kennzeichnungs-Lesegerät umfasst, das so ausgebildet ist, dass es eine Proben-ID der biologischen Probe bestimmt, indem es eine mit dem Probenröhrchen in Verbindung stehende Probenröhrchen-Kennzeichnung liest, und ferner so ausgebildet ist, dass es die Proben-ID der biologischen Probe an die Steuereinheit (20) übermittelt;
- eine Steuereinheit (20), die ausgebildet ist:
- zu bestimmen, ob dem Laborsystem (1) ein konkreter Prüfauftrag (100) vorliegt, der der Proben-ID der aufgenommenen biologischen Probe entspricht, wobei der konkrete Prüfauftrag (100) einen oder mehrere Verarbeitungsschritt(e), die an der biologischen Probe auszuführen sind, angibt;
- wenn dem Laborsystem (1) ein konkreter Prüfauftrag (100) vorliegt, der der aufgenommenen biologischen Probe entspricht:
- Abrufen des entsprechenden konkreten Prüfauftrags (100) und Veranlassen des Laborsystems (1), den einen oder die mehreren Verarbeitungsschritte an der biologischen Probe auszuführen;
- wenn dem Laborsystem (1) kein konkreter Prüfauftrag (100) vorliegt, der der aufgenommenen biologischen Probe entspricht:
- Erzeugen eines provisorischen Prüfauftrags (101), der der Proben-ID der aufgenommenen biologischen Probe entspricht, wobei der provisorische Prüfauftrag (101) einen oder mehrere provisorische Verarbeitungsschritte, die an der biologischen Probe auszuführen sind, angibt; und
- Veranlassen des Laborsystems (1), den einen oder die mehreren provisorischen Verarbeitungsschritte an der einen oder den mehreren biologischen Proben gemäß dem provisorischen Prüfauftrag (101) auszuführen;
**dadurch gekennzeichnet, dass**
- wenn dem Laborsystem (1) kein konkreter Prüfauftrag (100) vorliegt, der der aufgenommenen biologischen Probe entspricht, und
- wenn nicht automatisch ein provisorischer Prüfauftrag (101) erzeugt werden kann, der der Proben-ID entspricht, umfasst der Schritt des Erzeugens des provisorischen Prüfauftrags (101) die Schritte:
- Bestimmen einer oder mehrerer gängigster analytischer Untersuchung(en), die von dem Laborsystem ausgeführt werden;
- Abrufen eines oder mehrerer Verarbeitungsschritte, die den gängigsten analytischen Untersuchung(en) entsprechen.

12. Laborsystem (1) nach Anspruch 11, wobei die Steuereinheit (20) ferner ausgebildet ist:
- periodisch zu bestimmen, ob dem Laborsystem (1) einen konkreten Prüfauftrag (100), der der biologischen Probe entspricht, vorgelegt wurde, und;
- den konkreten Prüfauftrag (100) abzurufen, wenn dem Laborsystem (1) ein konkreter Prüfauftrag (100), der der biologischen Probe entspricht, vorgelegt wurde, und die Verarbeitungsschritt(e) des konkreten Prüfauftrags (100) und die provisorischen Verarbeitungsschritt(e) des provisorischen Prüfauftrags (101) zu vereinigen.

13. Laborsystem (1) nach Anspruch 12, wobei die Steuereinheit (20) ferner ausgebildet ist:
- einen oder mehrere ausgeführte provisorische Verarbeitungsschritte des provisorischen Prüfauftrags (101) zu bestimmen, die bereits von dem Laborsystem (1) ausgeführt worden sind;
- einen oder mehrere restliche Verarbeitungsschritte des konkreten Prüfauftrags (100) ohne entsprechende ausgeführte provisorische Verarbeitungsschritte zu bestimmen;
- das Laborsystem (1) zu veranlassen, die Ausführung noch nicht ausgeführter provisorischer Verarbeitungsschritte des provisorischen Prüfauftrags (101) aufzuheben;
- das Laborsystem (1) zu veranlassen, den einen oder die mehreren restlichen Verarbeitungsschritte des konkreten Prüfauftrags (100) an der biologischen Probe auszuführen.

14. Laborsystem (1) nach Anspruch 12 oder 13, wobei die Steuereinheit (20) ferner so ausgebildet ist, dass sie einen Alarm erzeugt, wenn einer oder mehrere der ausgeführten provisorischen Verarbeitungsschritte des provisorischen Prüfauftrags (101), die von dem Laborsystem (1) an der biologischen Probe ausgeführt werden, mit einem oder mehreren Verarbeitungsschritten des konkreten Prüfauftrags (100) in Konflikt stehen.

15. Laborsystem (1) nach einem der Ansprüche 11 bis 14, wobei die Steuereinheit (20) ferner so ausgebildet ist, dass sie einen provisorischen Prüfauftrag erzeugt, indem sie die Schritte durchführt:
- Abgleichen einer Vorlage mit der Probenröhrchen-Kennzeichnung und Abrufen des einen oder der mehreren Verarbeitungsschritte, die an der biologischen Probe durchzuführen sind, die mithilfe der Probenröhrchen-Kennzeichnung, die der Vorlage gleicht, identifiziert worden ist; und/oder
- Bestimmen der Röhrchenart des Probenröhrchens, das die biologische Probe enthält, und Abrufen des einen oder der mehreren Verarbeitungsschritte, die an biologischen Proben, die in Probenröhrchen der bestimmten Röhrchenart aufgenommen wurden, durchzuführen sind; und/oder
- Identifizieren eines Intervalls, zu dem die Probenröhrchen-Kennzeichnung gehört, und Abrufen des einen oder der mehreren Verarbeitungsschritte, die an biologischen Proben, die mithilfe der Probenröhrchen-Kennzeichnungen innerhalb des identifizierten Intervalls identifiziert worden sind, durchzuführen sind.

16. Laborsystem (1) nach einem der Ansprüche 11 bis 15, wobei die Probenzufuhrstation (12) in einem prä-analytischen Laborapparat (10pre) umfasst ist und/oder die Probenzufuhrstation (12) in einem oder mehreren Laborapparaten (10) umfasst ist; und/oder die Probenzufuhrstation (12) ein zweckbestimmter Laborapparat zum Beschicken von Proben (101d) ist.

17. Laborsystem (1) nach einem der Ansprüche 11 bis 15, wobei die Probenzufuhrstation (12) und der eine oder die mehreren Laborapparate (10) durch ein Probenröhrchen-Transportsystem verbunden sind, das ausgebildet ist, Probenröhrchen zwischen der Probenzufuhrstation (12) und dem einen oder den mehreren Laborapparaten (10) zu transportieren.

## Revendications

1. Procédé de fonctionnement d'un système de laboratoire (1) comprenant un ou plusieurs instrument(s) de laboratoire (10), une station d'entrée d'échantillon (12) et une unité de commande (20) reliés de façon communicative par un réseau de communication (70), le procédé comprenant les étapes de :
- réception d'un échantillon biologique par la station d'entrée d'échantillon (12), l'échantillon biologique étant contenu dans un tube d'échantillon ayant un identifiant de tube d'échantillon ;
- la station d'entrée d'échantillon (12) déterminant un ID d'échantillon de l'échantillon biologique en lisant l'identifiant de tube d'échantillon du tube d'échantillon ;
- la station d'entrée d'échantillon (12) transmettant l'ID d'échantillon de l'échantillon biologique à l'unité de commande (20) ;
- l'unité de commande (20) déterminant si un ordre de test défini (100) est disponible pour le système de laboratoire (1) correspondant à l'ID d'échantillon de l'échantillon biologique reçu, l'ordre de test défini (100) étant indicatif d'une ou plusieurs étape(s) de traitement à exécuter sur l'échantillon biologique ;
- si un ordre de test défini (100) correspondant à l'échantillon biologique reçu est disponible pour le système de laboratoire :
- l'unité de commande (20) récupérant l'ordre de test défini (100) correspondant et donnant l'ordre au système de laboratoire (1) d'exécuter la ou les étapes de traitement sur l'échantillon biologique ;
- si un ordre de test défini (100) correspondant à l'échantillon biologique reçu n'est pas disponible pour le système de laboratoire (1) :
- génération d'un ordre de test provisoire (101) correspondant à l'ID d'échantillon de l'échantillon biologique reçu, l'ordre de test provisoire (101) étant indicatif d'une ou plusieurs étapes de traitement provisoires à exécuter sur l'échantillon biologique ; et
- l'unité de commande (20) donnant l'ordre au système de laboratoire (1) d'exécuter la ou les étapes de traitement provisoires sur le ou les échantillons biologiques selon l'ordre de test provisoire (101) ;
**caractérisé en ce que**
- si un ordre de test défini (100) correspondant à l'échantillon biologique reçu n'est pas disponible pour le système de laboratoire (1) et
- si un ordre de test provisoire (101) correspondant à l'ID d'échantillon ne peut pas être généré automatiquement, l'étape de génération d'ordre de test provisoire (101) comprend les étapes de :
- détermination d'un ou plusieurs test(s) analytique(s) le(s) plus courant(s) exécuté(s) par le système de laboratoire ;
- récupération d'une ou plusieurs étapes de traitement correspondant au(x) test(s) analytique(s) le(s) plus courant(s).

2. Procédé de fonctionnement d'un système de laboratoire (1) selon la revendication 1, comprenant en outre les étapes de :
- après avoir donné l'ordre au système de laboratoire (1) d'exécuter la ou les étapes de traitement provisoires, l'unité de commande (20) déterminant de manière périodique si un ordre de test défini (100) correspondant à l'échantillon biologique est devenu disponible pour le système de laboratoire (1) ;
- si un ordre de test défini (100) correspondant à l'échantillon biologique est devenu disponible pour le système de laboratoire (1), l'unité de commande (20) récupérant l'ordre de test défini (100) ; et
- l'unité de commande (20) consolidant la ou des étapes de traitement de l'ordre de test défini (100) et la ou les étapes de traitement provisoires de l'ordre de test provisoire (101)
si un ordre de test défini (100) correspondant à l'échantillon biologique n'était pas disponible lors de la réception de l'échantillon biologique par la station d'entrée d'échantillon (12).

3. Procédé de fonctionnement d'un système de laboratoire (1) selon la revendication 2, dans lequel l'étape de consolidation de la ou des étapes de traitement de l'ordre de test défini (100) et de l'ordre de test provisoire (101) comprend :
- la détermination d'une ou plusieurs étapes de traitement provisoires exécutées de l'ordre de test provisoire (101) qui ont déjà été exécutées par le système de laboratoire (1) ;
- la détermination d'une ou plusieurs étapes de traitement restantes de l'ordre de test défini (100) sans aucune étape de traitement provisoire exécutée correspondante,
le procédé comprenant en outre les étapes de :
- si les étapes de traitement provisoires de l'ordre de test provisoire (101) qui ne sont pas encore exécutées entrent en conflit avec les étapes de traitement restantes de l'ordre de test défini (100), l'unité de commande (20) donnant l'ordre au système de laboratoire (1) d'annuler l'exécution des étapes de traitement provisoires de l'ordre de test provisoire (101) qui ne sont pas encore exécutées et d'exécuter la ou les étapes de traitement restantes de l'ordre de test défini (100) sur l'échantillon biologique.

4. Procédé de fonctionnement d'un système de laboratoire (1) selon l'une des revendications précédentes, dans lequel l'unité de commande (20) donne l'ordre à l'instrument de laboratoire (10) de tamponner l'échantillon biologique après exécution de la ou des étapes de traitement provisoires de l'ordre de test provisoire (101) et de récupérer l'échantillon biologique lorsque l'ordre de test défini (100) devient disponible.

5. Procédé de fonctionnement d'un système de laboratoire (1) selon la revendication 3 ou 4, comprenant en outre l'étape de déclenchement d'une alerte si une ou plusieurs des étapes de traitement provisoires exécutées de l'ordre de test provisoire (101) exécutées par le système de laboratoire (1) sur l'échantillon biologique entrent en conflit avec une ou plusieurs étapes de traitement de l'ordre de test défini (100).

6. Procédé de fonctionnement d'un système de laboratoire (1) selon l'une des revendications précédentes, dans lequel l'étape de génération de l'ordre de test provisoire (101) comprend les étapes de :
- mise en correspondance d'un modèle avec l'identifiant de tube d'échantillon ; et
- récupération d'une ou plusieurs étapes de traitement à effectuer sur des échantillons biologiques identifiés par un identifiant de tube d'échantillon correspondant au modèle.

7. Procédé de fonctionnement d'un système de laboratoire (1) selon l'une des revendications précédentes, dans lequel l'étape de génération de l'ordre de test provisoire (101) comprend les étapes de :
- identification d'un intervalle auquel appartient l'identifiant de tube d'échantillon ; et
- récupération d'une ou plusieurs étapes de traitement à effectuer sur des échantillons biologiques identifiés par un identifiant de tube d'échantillon dans l'intervalle identifié.

8. Procédé de fonctionnement d'un système de laboratoire (1) selon l'une des revendications précédentes, dans lequel l'étape de génération de l'ordre de test provisoire (101) comprend les étapes de :
- détermination du type de tube du tube d'échantillon qui contient ledit un ou plusieurs échantillons biologiques ; et
- récupération d'une ou plusieurs étapes de traitement à effectuer sur des échantillons biologiques reçus dans des tubes d'échantillons du type de tube déterminé.

9. Procédé de fonctionnement d'un système de laboratoire (1) selon l'une des revendications précédentes, comprenant en outre une ou plusieurs des étapes :
- l'unité de commande (20) demandant un ordre de test (100, 101) d'un système hôte (30), tel qu'un système d'information de laboratoire ;
- le système hôte (30) générant l'ordre de test provisoire (101) ;
- le système hôte (30) transmettant l'ordre de test provisoire (101) à l'unité de commande (20) ;
- le système hôte (30) recevant l'ordre de test défini (101) d'un opérateur via une interface utilisateur ;
- le système hôte (30) transmettant l'ordre de test défini (100) à l'unité de commande (20).

10. Procédé de fonctionnement d'un système de laboratoire (1) selon l'une des revendications précédentes, dans lequel les étapes de traitement provisoires de l'ordre de test provisoire (101) sont exécutées sur une aliquote de l'échantillon biologique et dans lequel toutes les étapes de traitement de l'ordre de test défini (100) sont effectuées sur l'échantillon biologique si une ou plusieurs des étapes de traitement provisoires exécutées de l'ordre de test provisoire (101) exécutées par le système de laboratoire (1) sur l'échantillon biologique entrent en conflit avec une ou plusieurs étapes de traitement de l'ordre de test défini (100).

11. Système de laboratoire (1) pour le traitement d'échantillon(s) biologique(s), le système de laboratoire (1) comprenant :
- un ou plusieurs instrument(s) de laboratoire (10) configuré(s) pour exécuter les étapes de traitement sur l'échantillon biologique,
- une station d'entrée d'échantillon (12) configurée pour recevoir un échantillon biologique compris dans un tube d'échantillon, la station d'entrée d'échantillon (12) comprenant un lecteur d'identifiant configuré pour déterminer un ID d'échantillon de l'échantillon biologique en lisant un identifiant de tube d'échantillon associé au tube d'échantillon et configuré en outre pour transmettre l'ID d'échantillon de l'échantillon biologique à l'unité de commande (20) ;
- une unité de commande (20) configurée pour :
- déterminer si un ordre de test défini (100) est disponible pour le système de laboratoire (1) correspondant à l'ID d'échantillon de l'échantillon biologique reçu, l'ordre de test défini (100) étant indicatif d'une ou plusieurs étape(s) de traitement à exécuter sur l'échantillon biologique ;
- si un ordre de test défini (100) correspondant à l'échantillon biologique reçu est disponible pour le système de laboratoire (1) :
- récupérer l'ordre de test défini (100) correspondant et donner l'ordre au système de laboratoire (1) d'exécuter la ou les étapes de traitement sur l'échantillon biologique ;
- si un ordre de test défini (100) correspondant à l'échantillon biologique reçu n'est pas disponible pour le système de laboratoire (1) :
générer un ordre de test provisoire (101) correspondant à l'ID d'échantillon de l'échantillon biologique reçu, l'ordre de test provisoire (101) étant indicatif d'une ou plusieurs étapes de traitement provisoires à exécuter sur l'échantillon biologique ; et
- donner l'ordre au système de laboratoire (1) d'exécuter la ou les étapes de traitement provisoires sur le ou les échantillons biologiques selon l'ordre de test provisoire (101) ;
**caractérisé en ce que**
- si un ordre de test défini (100) correspondant à l'échantillon biologique reçu n'est pas disponible pour le système de laboratoire (1) et
- si un ordre de test provisoire (101) correspondant à l'ID d'échantillon ne peut être généré automatiquement, l'étape de génération de l'ordre de test provisoire (101) comprend les étapes de :
- détermination d'un ou plusieurs test(s) analytique(s) le(s) plus courant(s) exécuté(s) par le système de laboratoire ;
- récupération d'une ou plusieurs étapes de traitement correspondant au(x) test(s) analytique(s) le(s) plus courant(s).

12. Système de laboratoire (1) selon la revendication 11, dans lequel l'unité de commande (20) est en outre configurée pour :
- déterminer périodiquement si un ordre de test défini (100) correspondant à l'échantillon biologique est devenu disponible pour le système de laboratoire (1) et ;
- récupérer l'ordre de test défini (100) si un ordre de test défini (100) correspondant à l'échantillon biologique est devenu disponible pour le système de laboratoire (1) et consolider la ou les étapes de traitement de l'ordre de test défini (100) et la ou les étapes de traitement provisoires de l'ordre de test provisoire (101).

13. Système de laboratoire (1) selon la revendication 12, dans lequel l'unité de commande (20) est en outre configurée pour :
- déterminer une ou plusieurs étapes de traitement provisoires exécutées de l'ordre de test provisoire (101) qui ont déjà été exécutées par le système de laboratoire (1) ;
- déterminer une ou plusieurs étapes de traitement restantes de l'ordre de test défini (100) sans étapes de traitement provisoires exécutées correspondantes ;
- donner l'ordre au système de laboratoire (1) d'annuler l'exécution des étapes de traitement provisoires de l'ordre de test provisoire (101) qui ne sont pas encore exécutées ;
- donner l'ordre au système de laboratoire (1) d'exécuter la ou les étapes de traitement restantes de l'ordre de test défini (100) sur l'échantillon biologique.

14. Système de laboratoire (1) selon la revendication 12 ou 13, dans lequel l'unité de commande (20) est en outre configurée pour générer une alerte si une ou plusieurs des étapes de traitement provisoires exécutées de l'ordre de test provisoire (101) exécutées par le système de laboratoire (1) sur l'échantillon biologique entrent en conflit avec une ou plusieurs étapes de traitement de l'ordre de test défini (100).

15. Système de laboratoire (1) selon l'une des revendications 11 à 14, dans lequel l'unité de commande (20) est en outre configurée pour générer un ordre de test provisoire en effectuant les étapes de :
- mise en correspondance d'un modèle avec l'identifiant de tube d'échantillon et récupérer la ou les étapes de traitement à effectuer sur l'échantillon biologique identifié par l'identifiant de tube d'échantillon correspondant au modèle ; et/ou
- détermination du type de tube du tube d'échantillon qui contient l'échantillon biologique et récupérer la ou les étapes de traitement à effectuer sur les échantillons biologiques reçus dans les tubes d'échantillon du type de tube déterminé ; et/ou
- identification d'un intervalle auquel appartient l'identifiant de tube d'échantillon et récupérer la ou les étapes de traitement à effectuer sur les échantillons biologiques identifiés par les identifiants de tube d'échantillon dans l'intervalle identifié.

16. Système de laboratoire (1) selon l'une des revendications 11 à 15, dans lequel la station d'entrée d'échantillon (12) est comprise dans un instrument de laboratoire pré-analytique (10pre), et/ou la station d'entrée d'échantillon (12) est comprise dans un ou plusieurs instruments de laboratoire (10) ; et/ou la station d'entrée d'échantillon (12) est un instrument de laboratoire dédié pour le chargement des échantillons (101d).

17. Système de laboratoire (1) selon l'une des revendications 11 à 15, dans lequel la station d'entrée d'échantillon (12) et le ou les instruments de laboratoire (10) sont reliés par un système de transport de tubes d'échantillon configuré pour transporter des tubes d'échantillon entre la station d'entrée d'échantillon (12) et le ou les instruments de laboratoire (10).
